# EUROPÄISCHE PATENTSCHRIFT

(11) **EP 1 143 918 B1**
(45) Veröffentlichungstag und Bekanntmachung des Hinweises auf die Patenterteilung: **20.08.2003**
(21) Anmeldenummer: 99963360.5
(22) Anmeldetag: 01.12.1999
(51) Int. Cl.: A61K 7/00

(54) **VERFAHREN ZUR HERSTELLUNG WÄSSRIGER GELE UND DEREN VERWENDUNG ZUR KÖRPERDEODORIERUNG**
METHOD FOR PRODUCING AQUEOUS GELS AND THE UTILISATION THEREOF FOR BODY DEODORISATION
PROCEDE DE PRODUCTION DE GELS AQUEUX ET LEUR UTILISATION COMME DEODORANTS CORPORELS

(30) Priorität: 11.12.1998 DE 19857235
(43) Veröffentlichungstag der Anmeldung: 17.10.2001
(73) Patentinhaber: Henkel Kommanditgesellschaft auf Aktien, 40589 Düsseldorf-Holthausen (DE)
(72) Erfinder: KROPF, Christian, D-40597 Düsseldorf (DE); FÖRSTER, Thomas, D-40699 Erkrath (DE); CLAAS, Marcus, D-40723 Hilden (DE); BANOWSKI, Bernhard, D-40597 Düsseldorf (DE); HELLER, Melita, D-40591 Düsseldorf (DE)
(86) Internationale Anmeldenummer: EP9909379
(87) Internationale Veröffentlichungsnummer: WO00035411

(56) Entgegenhaltungen:
- EP-A- 0 191 628
- EP-A- 0 518 175
- DE-A- 4 136 512
- DE-A- 4 329 129
- ERIC JORGE, THIERRY CHARTIER: "Ultrasonic Dispersion of Ceramic Powders" JOURNAL OF THE AMERICAN CERAMIC SOCIETY, vol. 73, no. 8, 1990, pages 2552-2554, XP000159709

## Beschreibung

Die Erfindung betrifft ein Verfahren zur Herstellung wäßriger Gele aus feinteiligen Oxiden, Oxidhydraten und Hydroxiden von Calcium, Magnesium, Aluminium, Titan, Zirkon oder Zink und die Verwendung dieser Gele zur Körperdeodorierung oder als Träger für andere kosmetische und dermatologische Wirkstoffe, insbesondere für Deodorant- und Antitranspirant-Wirkstoffe.

In der EP-A-0 518 175 wird ein Verfahren zur Herstellung eines neutralen Metalloxidsols, beispielsweise eines Titanoxidsols, beschrieben, welches durch Hydroxycarbonsäuren bzw. deren Derivate stabilisiert wird. Die Metalloxidsole, insbesondere die Titanoxidsole, werden bevorzugt in der Kosmetik eingesetzt.

Aus der DE-A-43 29 129 ist die Herstellung von Titanoxidsolen, die mit Hydroxycarbonsäuren stabilisiert sind, bekannt. Femer wird der Einsatz des durch das Verfahren erhaltenen Titanoxidsols in der Kosmetik, insbesondere als UV-Schutz, offenbart.

Für die Herstellung gelförmiger wäßriger Zubereitungen sind verschiedene Verfahren bekannt. Sie beruhen darauf, daß ein gelbildender Stoff, der entweder ein wasserlösliches Polymer oder ein mizellbildender amphiphiler Stoff ist, in Wasser gelöst wird. Je nach Konzentration des Gelbildners kann das Gel als Flüssigkeit mit nicht newtonischer Rheologie, meist als strukturviskose oder thixotrope Flüssigkeit oder als steifes, nicht fließendes Gel vorliegen, das sich z.B. zur Herstellung kosmetischer Stiftpräparate eignet.

Übliche Deodorant-Stifte werden z.B. auf Basis von wäßrigen Lösungen niederer Alkohole oder Polyole, darin gelösten Deodorant-Wirkstoffen und Seifen, z.B. Kalium- oder Natriumstearat als Gelbildner hergestellt. Aufgrund der Alkalinität der Seife können saure Wirkstoffe, z.B. Aluminiumhydroxychlorid in solche Träger nicht eingearbeitet werden.

Wegen dieser Nachteile wurden auch seifenfreie Antitranspirant-Stifte entwickelt, z.B. auf Basis flüssiger Öle, die mit Wachsen verdickt sind. Als flüssige Öle werden gern flüchtige Silikonöle verwendet, die eine Kühlwirkung auf der Haut ausüben. Solche Stiftmassen sind aber verhältnismäßig teuer und weisen auch Nachteile bei der stabilen Einarbeitung wasserlöslicher Wirkstoffe auf. Schließlich wurden auch Wasser-in-Öl-Emulsionen, bevorzugt auf Basis von flüchtigen Silikonölen, die bei nahezu gleichem Brechungsindex der wäßrigen und der öligen Phase transparent erscheinen, als Träger für Deodorant-Wirkstoffe vorgeschlagen.

Die vorliegende Erfindung betrifft ein völlig neues Verfahren zur Herstellung wäßriger Gele. Es beruht auf der Beobachtung, daß bestimmte Metalloxide in Form von mikrofeinen Pulvern nach Modifikation der Oberfläche unter Bindung von organischen Carbonsäuren in der Lage sind, gelförmige Dispersionen in Wasser auszubilden.

Gegenstand der Erfindung ist daher ein Verfahren zur Herstellung von wäßrigen Gelen aus feinteiligen Oxiden, Oxidhydraten oder Hydroxiden des Calciums, Magnesiums, Titans, Zirkoniums oder des Zinks, bei dem man die Oxide, Oxidhydrate oder Hydroxide mit einer Teilchengröße von weniger als 200 nm mit wäßrigen Lösungen von Carbonsäuren oder Hydroxycarbonsäuren mit 2 - 8 C-Atomen bei der Siedetemperatur des Wassers unter Normaldruck oder oberhalb von 100 °C unter Anwendung von Druck behandelt und die dabei erhaltenen modifizierten Pulver in Wasser oder in wäßrigen Zubereitungen dispergiert.

Geeignete feinteilige Oxide sind z.B. Magnesiumoxid, Aluminiumoxid, Titandioxid, Zirkondioxid und Zinkoxid. Ein geeignetes Oxidhydrat ist z.B. Aluminiumoxidhydrat (Böhmit) und geeignete Hydroxide sind z.B. Calciumhydroxid und Aluminiumhydroxid.

Die Teilchengröße dieser Oxide, Oxidhydrate und Hydroxide liegt unter 200 nm (Nanometer), wobei der Wert sich auf den Teilchendurchmesser in der Längsrichtung, d.h. in der Richtung der größeren Ausdehnung der Teilchen bezieht. Solche feinteiligen Oxide, Oxidhydrate oder Hydroxide lassen sich nach bekannten Verfahren, z.B. nach EP 711 217 A1 (Nanophase Technologies Corp.) herstellen. Geeignete Oxide sind unter dem Warenzeichen Nano Tek® im Handel. Auch durch Hydrolyse metallorganischer Verbindungen sind Oxidhydrate und Hydroxide in sehr feiner Verteilung zugänglich.

Bevorzugt geeignet für das erfindungsgemäße Verfahren sind Oxide, Oxidhydrate und Hydroxide mit einer Teilchengröße von weniger als 100 nm, bevorzugt von 1 - 50 nm.

Ein besonders bevorzugt geeignetes Nanopulver für das Verfahren der vorliegenden Erfindung ist ein Aluminiumoxidhydrat der Zusammensetzung AlOOH · H₂O (Böhmit) und einer spezifischen Oberfläche von mehr als 200 m²/g. Dieses Material ist preiswert in großen Mengen verfügbar. Eine geringfügige Verunreinigung mit ca. 4 - 14 Gew.-% Aluminium-Acetat-Hydrat (Al(OCOCH₃)₃ · H₂O) ist für die Zwecke der Erfindung nicht nachteilig. Das Produkt ist unter der Bezeichnung Disperal Sol P3 (Condea Chemie GmbH) im Handel.

Als wäßrige Carbonsäuren zur Oberflächenmodifikation der Oxid-Nanopartikel eignen sich alle ein- und mehrbasischen Carbonsäuren mit 2 - 8 C-Atomen, also z.B. Essigsäure, Propionsäure, Oxalsäure, Glutarsäure, Maleinsäure, Bernsteinsäure, Phthalsäure, Adipinsäure, Korksäure. Bevorzugt geeignet sind die Hydroxycarbonsäuren und Fruchtsäuren wie z.B. Glycolsäure, Milchsäure, Zitronensäure, Äpfelsäure, Weinsäure und Gluconsäure. Besonders bevorzugt wird als Carbonsäure eine Hydroxycarbonsäure aus der Gruppe Milchsäure, Zitronensäure, Äpfelsäure und Weinsäure eingesetzt.

Die Behandlung der Nanopulver mit der wäßrigen Lösung einer Carbon- oder Hydroxycarbonsäure erfolgt bevorzugt in der Weise, daß die feinteiligen Oxide, Oxidhydrate oder Hydroxide mit einer Lösung von 0,05 bis 0,5 Mol der Carbonsäure pro Mol des Oxids, Oxidhydrats oder Hydroxids behandelt werden. Diese Behandlung erfolgt bevorzugt über einen Zeitraum von 1 - 24 Stunden bei der Siedetemperatur des Wassers bei Normaldruck (100° C). Bei Anwendung von Druck kann die Behandlung auch bei Temperaturen oberhalb 100° C in entsprechend kürzerer Zeit erfolgen.

Durch die Behandlung mit den Carbonsäuren oder Hydroxycarbonsäuren wird die Oberfläche der Oxid- oder Hydroxy-Nanopartikel modifiziert Die Erfinder nehmen an, daß die Carbonsäuren oder Hydroxycarbonsäuren esterartig an die Oberfläche der Nanopartikel gebunden werden.

Die modifizierten Metalloxide, Oxidhydrate oder Hydroxide können entweder in Form der bei der Behandlung mit Carbonsäuren erhaltenen wäßrigen Dispersion oder nach vorheriger Isolierung zu wäßrigen Gelen weiterverarbeitet werden.

Das modifizierte Metalloxid-, Oxidhydrat- oder Hydroxid-Pulver wird aus dem Reaktionsgemisch bevorzugt durch Entwässerung isoliert. Zu diesem Zweck wird die Dispersion der Gefriertrocknung unterworfen. Dabei wird das Lösungsmittel bei tiefer Temperatur im Hochvakuum absublimiert.

Nach diesem Verfahren modifizierte Nanopulver enthalten zwischen 1 und 30 Gew.-%, bevorzugt zwischen 5 und 20 Gew.-%, des organischen Modifizierungsmittels. Ein weiterer Erfindungsgegenstand sind diese als Zwischenprodukte des erfindungsgemäßen Verfahrens hergestellten Oxide, Oxidhydrate oder Hydroxide von Calcium, Magnesium, Aluminium, Titan, Zirkon oder Zink, die eine Teilchengröße von weniger als 200 nm, bevorzugt von weniger als 100 nm, aufweisen und an der Oberfläche gebundene Carbonsäuren oder Hydroxycarbonsäuren in einer Menge von 1 - 30 Gew.-%, bezogen auf das wasserfreie Produkt, enthalten.

Die entwässerten, modifizierten Oxid-, Oxidhydrat- oder Hydroxid-Nanopulver werden zur Herstellung der Gele in Wasser oder in einer wäßrigen Zubereitung dispergiert. Diese Dispergierung erfordert zur Ausbildung der Gelstruktur eine gewisse Hydratationszeit. Diese Hydratation kann durch mechanische Energiezufuhr, z.B. durch Rühren oder Homogenisieren unter Zuhilfenahme geeigneter Rühr-, Misch- und Homogenisiereinrichtungen, z.B. von Kolloid-Mühlen, Hochdruck-Homogenisatoren oder Ultraschall-Dispergatoren beschleunigt werden.

In einer besonderen Ausführungsform des erfindungsgemäßen Verfahrens wird das modifizierte Pulver in Wasser oder wäßrigen Zubereitungen unter Anwendung von Ultraschall dispergiert und die Dispersion durch Zugabe von Alkalien auf einen pH-Wert von 4 bis 7 eingestellt.

Geeignete Alkalien sind vor allem wäßrige Lösungen von Alkalihydroxiden oder Alkalicarbonaten, z.B. Natriumhydroxid oder Kaliumhydroxid. Geeignet ist aber auch wäßriges Ammoniak sowie Mono-, Di- oder Trialkanolamine mit Alkanolgruppen mit 2 - 4 C-Atomen.

Während der Hydratation nimmt die Viskosität der Dispersion allmählich zu und kann bei einem Gehalt von mehr als 10 Gew.-% der modifizierten Nanopulver zu einem festen Gel führen, das sich für kosmetische Stiftzubereitungen eignet.

Die erfindungsgemäß hergestellten wäßrigen Gele enthalten die feinteiligen Metalloxide, Oxidhydrate oder Hydroxide in einer Teilchengröße, die die Transparenz dieser Gele kaum beeinträchtigt. Die feinteiligen Metalloxide, Oxidhydrate oder Hydroxide weisen darüber hinaus eine geruchsabsorbierende Wirkung auf. Die nach dem erfindungsgemäßen Verfahren erhältlichen wäßrigen Gele eignen sich daher zur Körperdesodorierung. Je nach Konzentration der feinteiligen Pulver und nach Viskosität der Gele können sie als Spray, Roll-On-Präparat, Deo-Creme oder Deodorant-Stift eingesetzt werden.

Für die Verwendung zur Körperdeodorierung setzt man den erfindungsgemäß erhältlichen Gelen bevorzugt noch weitere Komponenten zu, die in kosmetischen Körperbehandlungsmitteln üblich und zweckmäßig sind. Solche Komponenten können z.B. Duftstoffe, hautweichmachende Ölkomponenten, entzündungshemmende Wirkstoffe, Farbstoffe, Antioxidantien, Komplexbildner sowie gegebenenfalls Tenside oder Emulgatoren zur stabilen Einarbeitung dieser Komponenten sein. Bei der Herstellung von Körperdeodorierungsmitteln kann man auch so vorgehen, daß man in eine wäßrige Zubereitung der genannten Komponenten die erfindungsgemäß modifizierten Oxid-, Oxidhydrat- oder Hydroxid-Nanopulver einträgt und zum Gel hydratisiert.

Die nach dem erfindungsgemäßen Verfahren erhältlichen Gele eignen sich natürlich auch als Träger für kosmetische und dermatologische Wirkstoffe aller Art, die in Form von mehr oder weniger fließfähigen, gelförmigen Zubereitungen oder von steifen Gelstiften auf die Haut oder das Haar aufgetragen werden. Solche Wirkstoffe können z.B. festigende oder avivierende Komponenten für das Haar, Haarfärbemittel oder haarbleichende Oxidationsmittel, haarverformende Reduktionsmittel oder Enthaarungsmittel sein.

Auch hautkosmetische Wirkstoffe wie z.B. Vitamine, Panthenol, Pflanzenextrakte, sebostatische, entzündungshemmende, UV-absorbierende oder antimikrobielle Wirkstoffe können in die erfindungsgemäß hergestellten wäßrigen Gele als Träger eingearbeitet werden.

Besonders bevorzugt eignen sich die erfindungsgemäß herstellbaren Gele aber als Träger für andere deodorierende und schweißhemmende Wirkstoffe. Ein besonders bevorzugter Patentgegenstand sind daher Körper-Deodorantien mit einem Gehalt an deodorierenden oder schweißhemmenden Wirkstoffen in einem wäßrigen, gelförmigen Träger, der nach dem erfindungsgemäßen Verfahren erhältlich ist.

Als deodorierende Wirkstoffe werden üblicherweise antimikrobielle Stoffe eingesetzt, die gegen die schweißzersetzenden Mikroben wirksam sind. Geeignete antimikrobielle Stoffe sind z.B. Chlorhexidin, Triclosan, Phenoxyethanol oder antimikrobielle ätherische Öle und Farnesol. Auch esterasehemmende Stoffe, die in den Stoffwechsel der schweißzersetzenden Mikroorganismen eingreifen, wie z.B. Triethylcitrat oder Triacetin, werden als deodorierende Komponenten verwendet. Als transpirationshemmende Stoffe werden meist adstringierende, eiweißkoagulierende Salze, z.B. Aluminiumhydroxychlorid, Alaune z.B. Kalium-aluminiumsulfat oder Natrium-Aluminium-chlorhydroxy-lactat eingesetzt.

Der gelförmige Träger kann dabei fließfähig sein oder die Konsistenz einer Creme oder die eines Stiftes aufweisen. Die Anwendung flüssiger Deodorantien erfolgt z.B. durch sogenannte Rollkugel-Auftragsgeräte oder durch Sprays, die entweder durch eine Pumpmechanik oder mit Hilfe von Treibgasen auf die Haut gebracht werden.

Erfindungsgemäße Deodorant- und Antitranspirant-Zubereitungen können darüber hinaus alle für solche Zubereitungen üblichen Hilfsmittel enthalten. Solche sind vor allem z.B. hautweichmachende Ölkomponenten oder Silikone und die zu deren Emulgierung erforderlichen oberflächenaktiven Substanzen. Besonders vorteilhaft lassen sich Ölkomponenten in Form von Mikroemulsionen in erfindungsgemäße Körperdeodorantien einarbeiten. Diese lassen sich nach bekannten Verfahren herstellen, wie sie z.B. aus DE 4417476 A1 oder aus DE 4411557 bekannt sind. Darüber hinaus können erfindungsgemäße Körperdeodorantien Duftstoffe, Farbstoffe, Komplexbildner, pH-Stellmittel, Antioxidantien und andere für diesen Zweck übliche Hilfsstoffe enthalten.

Die folgenden Beispiele sollen den Erfindungsgegenstand näher erläutern:

### 1. Herstellung eines Al-Oxidhydrat-Gels

### 1a Oberflächenmodifikation

20,4 g einer 85 %igen wäßrigen Milchsäure-Lösung wurden in 797 g Wasser (vollentsalzt) gelöst, darin wurden 120 g Dispersal Sol P3 (Böhmit-Pulver) dispergiert und die Dispersion unter Rühren und Rückflußkühlung 45 Minuten zum Sieden erhitzt. Dabei erfolgte eine Modifikation der Partikeloberfläche durch gebundene Lactatgruppen. Die Rührleistung wurde mit zunehmender Viskosität des Systems erhöht. Nach dem Abkühlen wurde die Dispersion durch Gefriertrocknung entwässert.

### 1b Dispergierung zum Gel

10 g des oberflächenmodifizierten Böhmit-Pulvers wurden in 40 g einer wäßrigen Natronlauge (pH = 11,3) unter Anwendung von Ultraschall eingearbeitet. Nach 24 Stunden Ruhezeit gelierte die Dispersion und es wurde ein Gel mit einem pH-Wert von 4,8 und einem Gehalt an stabil dispergiertem Böhmit von 20 Gew.-% erhalten. Die Teilchengrößenverteilung erstreckte sich von 5 bis 45 nm (d₁₀ = 9,4 nm, d₅₀ = 12 nm, d₉₀ = 19,3 nm).

### 2. Herstellung eines ZnO-Gels

### 2a Oberflächenmodifikation

7,8 g einer 85 %igen wäßrigen Milchsäurelösung wurden in 690 g Wasser (vollentsalzt) gelöst, darin wurden 60 g ZnO (spez. Oberfläche 80 m²/g) dispergiert und die Dispersion unter Rühren und Rückflußkühlung 45 Minuten zum Sieden erhitzt. Mit zunehmender Viskosität der Dispersion wurde die Rührleistung erhöht. Nach dem Abkühlen wurde die Dispersion gefriergetrocknet.

### 2b Dispergierung zum Gel

5 g des gefriergetrockneten Pulvers wurde in 20 g einer verdünnten Natronlauge (pH = 11,3) unter Anwendung von Ultraschall eingearbeitet. Es wurde eine gelartige Dispersion mit einem pH-Wert von 4,2 erhalten. Die Teilchengrößenverteilung der ZnO-Partikel erstreckte sich von 25 nm bis 85 nm (d₉₀ = 57,2 nm).

### 3. Herstellung eines TiO₂-Gels

### 3a Oberflächenmodifikation

3,0 g Zitronensäure wurden in 416 g Wasser (vollentsalzt) gelöst. Darin wurden 21,9 g TiO₂ (spez. Oberfläche 160 m²/g) dispergiert und die Dispersion unter Rühren und Rückflußkühlung 45 Minuten zum Sieden erhitzt. Mit zunehmender Viskosität der Dispersion wurde die Rührleistung erhöht. Nach dem Abkühlen wurde die Dispersion durch Gefriertrocknung entwässert.

### 3b Dispergierung zum Gel

5 g des entwässerten Produktes wurden in 15 g Wasser (vollentsalzt) dispergiert, der pH-Wert wurde mit 0,1 M Natronlauge auf 4,2 eingestellt. Die Dispersion wurde zunächst mit Ultraschall behandelt; nach 24 h Ruhezeit wurde eine gelartige Dispersion mit einer Teilchengrößenverteilung von 5 nm bis 39 nm (d₉₀ = 24,7 nm) erhalten.

### 4. Herstellung eines ZrO₂-Gels

### 4a Oberflächenmodifikation

7,0 g einer 85 %igen wäßrigen Milchsäurelösung wurden in 600 g Wasser (vollentsalzt) gelöst und in dieser Lösung 53,5 g Zirkondioxid (spez. Oberfläche 130 m²/g) dispergiert. Diese Dispersion wurde 45 Minuten unter Rühren und Rückflußkühlung zum Sieden erhitzt. Mit zunehmender Viskosität der Dispersion wurde die Rührleistung erhöht. Nach dem Abkühlen wurde die Dispersion durch Gefriertrocknung entwässert.

### 4b Dispergierung zum Gel

5 g des entwässerten Produktes wurden in 15 g Wasser (vollentsalzt) dispergiert, der pH-Wert mit 0,1 M Natronlauge auf 4,2 eingestellt und die Dispersion dann mit Ultraschall behandelt. Nach 24 h Ruhezeit wurde ein Gel erhalten, welches 25 Gew.-% dispergierte ZrO₂-Teilchen mit einer Teilchengrößenverteilung von 6 nm bis 52 nm (d₉₀ = 21,5 nm) enthielt.

### 5. Herstellung von Antitranspirant-Gelen

### Herstellungsweise

Modifiziertes Böhmit-Pulver (gemäß Beispiel 1a) wurde in Wasser dispergiert, dann wurde eine 50 %ige wäßrige Lösung von Aluminiumhydroxychlorid zugesetzt und das Gemisch unter Einwirkung von Ultraschall in ein Gel überführt.
Rezepturen (%-Angaben beziehen sich auf Aktivsubstanz (AS)).

| | **5.1** | **5.2** | **5.3** |
|---|---|---|---|
| Modifiziertes Böhmit-Pulver (Beispiel 1b) | 7 Gew.-% | 8 Gew.-% | 13 Gew.-% |
| Aluminium-hydroxychlorid (100 % Aktivsubstanz) | 20 Gew.-% | 20 Gew.-% | 20 Gew.-% |
| Wasser | 73 Gew.-% | 72 Gew.-% | 67 Gew.-% |
| pH-Wert | 4,3 | 4,3 | 4,2 |
| Gelstruktur | thixotrop | thixotrop | nicht thixotrop |

### 6. Antitranspirant Roll-On-Formulierung

| | **6.1** | **6.2** | **6.3** |
|---|---|---|---|
| APG 600 | 0,050 Gew.-% | 0,050 Gew.-% | 0,050 Gew.-% |
| APG 220 | 0,150 Gew.-% | 0,150 Gew.-% | 0,150 Gew.-% |
| Cetiol OE | 0,030 Gew.-% | 0,030 Gew.-% | 0,030 Gew.-% |
| Eutanol G | 0,007 Gew.-% | 0,007 Gew.-% | 0,007 Gew.-% |
| Citronensäure (krist.) | 0,005 Gew.-% | 0,005 Gew.-% | 0,005 Gew.-% |
| Parfüm | 0,300 Gew.-% | 0,300 Gew.-% | 0,300 Gew.-% |
| Aluminiumhydroxychlorid | 20,000 Gew.-% | 20,000 Gew.-% | 20,000 Gew.-% |
| mod. Böhmit (Beispiel 1b) | 4,000 Gew.-% | 6,000 Gew.-% | 6,000 Gew.-% |
| Wasser | 75,460 Gew.-% | 73,460 Gew.-% | 76,460 Gew.-% |

Es wurden die folgenden Handelsprodukte verwendet:

| | |
|---|---|
| Disperal Sol P3 (Condea) | Zubereitung aus 93,8 Gew.-% AlOOH · H₂O und 6,2 Gew.-% Al(CH₃COO)₃ · H₂O Spez. Oberfläche: ca. 331 m²/g Kornverteilung: < 25 µm : 8,7 Gew.-% < 45 µm : 18,0 Gew.-% < 90 µm : 55,7 Gew.-% |
| | |
| Aluminiumhydroxychlorid | Locron L (Clariant) 50 %ige wäßrige Lösung |
| | |
| APG 600 | Plantacare 1200 UP (Henkel KGaA) Aktivsubstanz : 50 - 53 Gew.-% Alkyl-C₁₂-C₁₆-oligo(1,4)-glucosid |
| | |
| APG 220 | Plantacare 220 UP (Henkel KGaA) Aktivsubstanz: 62 - 65 Gew.-% Alkyl-C₈-C₁₀-oligo(1,5)-glucosid |

## Patentansprüche

1. Verfahren zur Herstellung von wäßrigen Gelen aus feinteiligen Oxiden, Oxidhydraten und Hydroxiden von Calcium, Magnesium, Aluminium, Titan, Zirkon oder Zink, **dadurch gekennzeichnet, daß** man die Oxide, Oxidhydrate oder Hydroxide mit einer Teilchengröße von weniger als 200 nm mit wäßrigen Lösungen von Carbonsäuren oder Hydroxycarbonsäuren mit 2 - 8 C-Atomen bei der Siedetemperatur des Wassers unter Normaldruck oder oberhalb von 100°C unter Anwendung von Druck behandelt und die dabei erhaltenen modifizierten Pulver in Wasser oder wäßrigen Zubereitungen dispergiert.

2. Verfahren nach Anspruch 1, **dadurch gekennzeichnet, daß** die feinteiligen Oxide, Oxidhydrate oder Hydroxide mit einer Lösung von 0,05 - 0,5 Mol der Carbonsäure pro Mol des Oxids, Oxidhydrats oder Hydroxids umgesetzt werden.

3. Verfahren nach Anspruch 1 oder 2, **dadurch gekennzeichnet, daß** als Oxidhydrat ein Aluminiumoxidhydrat der Zusammensetzung AlO(OH) H₂O (Böhmit) und einer spezifischen Oberfläche von mehr als 200 m²/g eingesetzt wird.

4. Verfahren nach einem der Ansprüche 1 - 3, **dadurch gekennzeichnet, daß** als Carbonsäure eine Hydroxycarbonsäure aus der Gruppe Milchsäure, Zitronensäure, Äpfelsäure, Weinsäure eingesetzt wird.

5. Verfahren nach einem der Ansprüche 1 - 4, **dadurch gekennzeichnet, daß** die modifizierten Pulver in Wasser unter Anwendung von Ultraschall dispergiert werden und die Dispersion durch Zugabe von Alkalien auf einen pH-Wert von 4 - 7 eingestellt wird.

6. Verwendung der Gele, die erhältlich sind nach dem Verfahren gemäß Anspruch 1 - 5 zur Körperdesodorierung.

7. Verwendung der Gele, die erhältlich sind nach dem Verfahren gemäß Anspruch 1 - 5 als Träger für kosmetische und dermatologische Wirkstoffe.

8. Feinteilige Oxide, Oxidhydrate und Hydroxide von Calcium, Magnesium, Aluminium, Titan, Zirkon oder Zink, **gekennzeichnet durch** eine Teilchengröße von weniger als 200 nm, bevorzugt von weniger als 100 nm, und **durch** an der Oberfläche gebundenen Carbonsäuren oder Hydroxycarbonsäuren mit 2 - 8 C-Atomen in einer Menge von 1 - 30 Gew.-%, bezogen auf das wasserfreie Produkt.

9. Körperdeodorantien mit einem Gehalt an deodorierenden oder schweißhemmenden Wirkstoffen in einem wäßrigen, gelförmigen Träger, **dadurch gekennzeichnet, daß** der gelförmige Träger erhältlich ist nach dem Verfahren gemäß Anspruch 1 - 5.

## Claims

1. A process for the production of water-containing gels from fine-particle oxides, hydrated oxides and hydroxides of calcium, magnesium, aluminium, titanium, zirconium or zinc, **characterized in that** the oxides, hydrated oxides or hydroxides with a particle size of less than 200 nm are treated with aqueous solutions of carboxylic acids or hydroxycarboxylic acids containing 2 to 8 carbon atoms at the boiling temperature of water under normal pressure or at a temperature above 100°C where pressure is applied and the modified powder obtained is dispersed in water or aqueous preparations.

2. A process as claimed in claim 1, **characterized in that** the fine-particle oxides, hydrated oxides or hydroxides are reacted with a solution of 0.05 to 0.5 mol of the carboxylic acid per mol of the oxide, oxide hydrate or hydroxide.

3. A process as claimed in claim 1 or 2, **characterized in that** a hydrated aluminium oxide with the composition AlO(OH)·H₂O (boehmite) and a specific surface of more than 200 m²/g is used as the hydrated oxide.

4. A process as claimed in any of claims 1 to 3, **characterized in that** a hydroxycarboxylic acid from the group consisting of lactic acid, citric acid, malic acid and tartaric aid is used as the carboxylic acid.

5. A process as claimed in any of claims 1 to 4, **characterized in that** the modified powder is dispersed in ultrasonicated water and the dispersion is adjusted to a pH of 4 to 7 by addition of alkalis.

6. The use of the gels obtainable by the process claimed in claims 1 to 5 for deodorizing the body.

7. The use of the gels obtainable by the process claimed in claims 1 to 5 as carriers for cosmetic and dermatological active ingredients.

8. Fine-particle oxides, hydrated oxides and hydroxides of calcium, magnesium, aluminium, titanium, zirconium or zinc, **characterized by** a particle size of less than 200 mm and preferably less than 100 nm and by carboxylic acids or hydroxycarboxylic acids with 2 to 8 carbon atoms fixed to the surface in a quantity of 1 to 30% by weight, based on the water-free product.

9. Body deodorants containing deodorizing or perspiration-inhibiting active ingredients in a water-containing gel-form carrier, **characterized in that** the gel-form carrier is obtainable by the process claimed in claims 1 to 5.

## Revendications

1. Procédé pour la préparation de gels aqueux à partir d'oxydes, d'hydrates d'oxydes et d'hydroxydes de calcium, de magnésium, d'aluminium, de titane, de zirconium ou de zinc finement divisés, **caractérisé en ce qu'**on traite les oxydes, les hydrates d'oxydes ou les hydroxydes possédant une granulométrie inférieure à 200 nm avec des solutions aqueuses d'acides carboxyliques ou d'acides hydroxy-carboxyliques contenant de 2 à 8 atomes de carbone à la température d'ébullition de l'eau sous pression normale ou à une température supérieure à 100 °C en appliquant de la pression et on disperse dans de l'eau ou dans des préparations aqueuses la poudre modifiée obtenue en l'occurrence.

2. Procédé selon la revendication 1, **caractérisé en ce qu'**on fait réagir les oxydes, les hydrates d'oxydes ou les hydroxydes finement divisés avec une solution de 0,05 - 0,5 mole de l'acide carboxylique par mole de l'oxyde, de l'hydrate d'oxyde ou de l'hydroxyde.

3. Procédé selon la revendication 1 ou 2, **caractérisé en ce qu'**on met en oeuvre, à titre d'hydrate d'oxyde, un hydrate d'oxyde d'aluminium de composition AIO(OH) · H₂O (boehmite) et possédant une surface spécifique supérieure à 200 m²/g.

4. Procédé selon l'une quelconque des revendications 1 à 3, **caractérisé en ce qu'**on met en oeuvre, à titre d'acide carboxylique, un acide hydroxycarboxylique choisi parmi le groupe comprenant l'acide lactique, l'acide citrique, l'acide malique, l'acide tartrique.

5. Procédé selon l'une quelconque des revendications 1 à 4, **caractérisé en ce qu'**on disperse la poudre modifiée dans de l'eau en appliquant des ultrasons et on règle la dispersion par addition d'alcalis à une valeur de pH de 4 à 7.

6. Utilisation des gels, que l'on obtient conformément au procédé selon les revendications 1 à 5, pour la désodorisation corporelle.

7. Utilisation des gels, que l'on obtient conformément au procédé selon les revendications 1 à 5, comme support pour des substances actives cosmétiques et dermatologiques.

8. Oxydes, hydrates d'oxydes et hydroxydes de calcium, de magnésium, d'aluminium, de titane, de zirconium ou de zinc finement divisés, **caractérisés par** une granulométrie inférieure à 200 nm, de préférence inférieure à 100 nm, et par des acides carboxyliques ou par des acides hydroxycarboxyliques contenant de 2 à 8 atomes de carbone, liés à la surface, en une quantité de 1 à 30 % en poids, rapportés au produit anhydre.

9. Déodorants corporels possédant une teneur en substances actives déodorantes ou inhibant la transpiration dans un support aqueux sous forme de gel, **caractérisés en ce que** le support sous forme de gel peut être obtenu conformément au procédé selon les revendications 1 à 5.
